# EUROPEAN PATENT SPECIFICATION

(11) **EP 3 531 129 B1**
(45) Date of publication and mention of the grant of the patent: **28.04.2021**
(21) Application number: 17862013.4
(22) Date of filing: 18.10.2017
(51) Int. Cl.: G01N 33/74, C07K 16/26

(54) **IMMUNOASSAY METHOD USING ANTI-HUMAN BNP FRAGMENT (4-32) ANTIBODY**
IMMUNTESTVERFAHREN UNTER VERWENDUNG EINES ANTI-HUMANEN BNP-FRAGMENT (4-32)-ANTIKÖRPERS
PROCÉDÉ D'IMMUNOESSAI UTILISANT UN ANTICORPS ANTI-FRAGMENT BNP HUMAIN (4-32)

(30) Priority: 18.10.2016 JP 2016204285
(43) Date of publication of application: 28.08.2019
(73) Proprietor: Sekisui Medical Co., Ltd., Tokyo 103-0027 (JP)
(72) Inventor: SHIMIZU, Tomo, Tokyo 103-0027 (JP); MIYAZAKI, Osamu, Tokyo 103-0027 (JP); SUZUKI, Toru, Tokyo 141-0021 (JP)
(74) Representative: Hoffmann Eitle
(86) International application number: PCT/JP2017/037763
(87) International publication number: WO 2018/074533

(56) References cited:
- WO-A1-2010/023749
- JP-A- 2009 514 975
- JP-A- 2014 091 719
- US-A1- 2011 151 583
- MAALOUF RITA ET AL: "A review on B-type natriuretic peptide monitoring: assays and biosensors", HEART FAILURE REVIEWS, KLUWER ACADEMIC PUBLISHERS, DORDRECHT, NL, vol. 21, no. 5, 15 March 2016 (2016-03-15) , pages 567-578, XP036031881, ISSN: 1382-4147, DOI: 10.1007/S10741-016-9544-9 [retrieved on 2016-03-15]
- H. FUJIMOTO ET AL: "Processed B-Type Natriuretic Peptide Is a Biomarker of Postinterventional Restenosis in Ischemic Heart Disease", CLINICAL CHEMISTRY, vol. 59, no. 9, 13 May 2013 (2013-05-13), pages 1330-1337, XP055442128, ISSN: 0009-9147, DOI: 10.1373/clinchem.2013.203406
- NAKAGAWA YASUAKI ET AL: "Atrial and brain natriuretic peptides: Hormones secreted from the heart", PEPTIDES, ELSEVIER, AMSTERDAM, NL, vol. 111, 31 May 2018 (2018-05-31), pages 18-25, XP085582009, ISSN: 0196-9781, DOI: 10.1016/J.PEPTIDES.2018.05.012
- TORU SUZUKI ET AL: "Prognostic role of Molecular Forms of B-type Natriuretic Peptide in acute Heart Failure", CLINICAL CHEMISTRY, vol. 63, no. 4, 1 April 2017 (2017-04-01), pages 880-886, XP055691286, ISSN: 0009-9147, DOI: 10.1373/clinchem.2016.265140

## Description

### TECHNICAL FIELD

The present invention relates to an immunassay and an immunoassay reagent for human BNP fragments. Specifically, the invention relates to an assay and an assay reagent for human BNP fragments using a specific anti-human BNP fragment (4-32) antibody.

### BACKGROUND ART

BNP, which stands for brain natriuretic peptide, is a 32-amino acid hormone synthesized in and secreted by the heart. The BNP concentration in blood is known to increase in response to increased heart load or myocardial hypertrophy. In clinical applications, BNP has widely been used as a biochemical marker of heart failure, and measurement of BNP levels has found its utility in screening of heart disease, severity evaluation of heart failure, and determination of therapeutic effect on heart failure, or as a prognostication index of cardiac arrest patients.

Patent Document 1 and Non-Patent Document 1 disclose methods that measure blood BNP for diagnosis of myocardial ischemic conditions. Specifically, the methods used specific antibodies to selectively concentrate BNP and its processed products (e.g., human BNP fragment (3-32), human BNP fragment (4-32), and human BNP fragment (5-32)) in blood samples, and subsequently, through detection of these processed products by mass spectrometry, found a correlation between ratios of specific processed products and the presence or absence of restenosis.

Patent Document 2 discloses a method for specifically assaying human BNP fragment (3-32) with the use of a human BNP (3-32)-specific antibody that reacts with human BNP fragment (3-32) but does not react with full-length human BNP and human proBNP. This patent document suggests that the method is potentially useful for diagnoses of diseases that use increase and decrease of human BNP (3-32) as an index, such as diagnoses of high-blood pressure and heart and renal functions.

### CITATION LIST

### PATENT DOCUMENT

Patent Document 1: WO2010/023749
Patent Document 2: JP 2014-91719 A

### NON-PATENT DOCUMENT

Non-Patent Document 1: Clinical Chemistry 59: 9 (2013), p.p. 1-8

### SUMMARY OF INVENTION

### TECHNICAL PROBLEM

The methods of Patent Document 1 and Non-Patent Document 1 use an antibody that reacts with all of human BNP (1-32), BNP (3-32), BNP (4-32), and BNP (5-32), and concentrate the BNP fragments in a sample. The fractions of these fragments are then calculated by mass spectrometry from their signal intensity ratios in a mass spectrometry spectrum. That is, these methods use a mass spectrometer to distinguish and detect BNP fragments, and this requires an extensive device configuration, and, accordingly, the procedures are complex. The methods are therefore unsuited for fast processing of a large number of specimens.

The method disclosed in Patent Document 2 is related to an antibody that reacts with human BNP fragment (3-32) but does not react with lull-length human BNP and human proBNP. However, this document does not disclose whether the antibody reacts with other fragments, human BNP (4-32) and BNP (5-32). That is, Patent Document 2 does not disclose whether the antibody specifically reacts only to human BNP fragment (3-32) among different human BNP fragments. This document also does not describe or indicate an antibody capable of specifically detecting fragments other than human BNP fragment (3-32).

That is, no method has been established that can measure BNP and BNP fragments solely by immunoassay.

The present invention is intended to enable detection of specific human BNP fragments solely by immunoassay, and, through measurement of ratios of the human BNP fragments, provides a convenient way of providing information useful for diagnosis and prognosis of heart disease. The invention achieves this by providing an assay that uses an antibody specific to human BNP fragment (4-32) - a fragment that has not caught much attention in the past.

### SOLUTION TO PROBLEM

In order to provide a solution to the foregoing problems, the present inventors conducted intensive studies to find an antibody that can specifically detect only human BNP fragment (4-32), and, for the first time, successfully obtained a plurality of such antibodies. The present invention has been completed after demonstrating that specific detection of human BNP fragment (4-32) in a sample is indeed possible with these antibodies.

Specifically, the present invention is configured as follows.
<1> An immunoassay method for human BNP fragment (4-32) in a sample, wherein the method uses an antibody that reacts with human BNP fragment (4-32) but does not react with full-length human BNP (1-32), human BNP fragment (3-32), and human BNP fragment (5-32).
<2> The immunoassay method according to item <1>, wherein the antibody that reacts with human BNP fragment (4-32) but does not react with full-length human BNP (1-32), human BNP fragment (3-32), and human BNP fragment (5-32) is an antibody that reacts with human BNP fragment (4-32) at a site containing the N-terminus.
<3> The immunoassay method according to item <1> or <2>, wherein the antibody that reacts with human BNP fragment (4-32) but does not react with full-length human BNP (1-32), human BNP fragment (3-32), and human BNP fragment (5-32) is an antibody that reacts with human BNP fragment (4-10).
<4> The immunoassay method according to any one of items <1> to <3>, wherein the antibody that reacts with human BNP fragment (4-32) but does not react with full-length human BNP (1-32), human BNP fragment (3-32), and human BNP fragment (5-32) is a monoclonal antibody.
<5> An immunoassay reagent for human BNP fragment (4-32), wherein the immunoassay reagent uses an antibody that reacts with human BNP fragment (4-32) but does not react with full-length human BNP (1-32), human BNP fragment (3-32), and human BNP fragment (5-32).
<6> The immunoassay reagent according to item <5>, wherein the antibody that reacts with human BNP fragment (4-32) but does not react with full-length human BNP (1-32), human BNP fragment (3-32), and human BNP fragment (5-32) is an antibody that reacts with human BNP fragment (4-32) at a site containing the N-terminus.
<7> The immunoassay reagent according to item <5> or <6>, wherein the antibody that reacts with human BNP fragment (4-32) but does not react with full-length human BNP (1-32), human BNP fragment (3-32), and human BNP fragment (5-32) is an antibody that reacts with human BNP fragment (4-10).
<8> The immunoassay reagent according to any one of items <5> to <7>, wherein the antibody that reacts with human BNP fragment (4-32) but does not react with full-length human BNP (1-32), human BNP fragment (3-32), and human BNP fragment (5-32) is a monoclonal antibody.
<9> An antibody that reacts with human BNP fragment (4-32) but does not react with full-length human BNP (1-32), human BNP fragment (3-32), and human BNP fragment (5-32).
<10> The antibody according to item <9>, which reacts with human BNP fragment (4-32) at a site containing the N-terminus.
<11> The antibody according to item <9> or <10>, which reacts with human BNP fragment (4-10).
<12> The antibody according to any one of items <9> to <11>, which is a monoclonal antibody.

### ADVANTAGEOUS EFFECTS OF INVENTION

The present invention has enabled a specific immunoassay of human BNP fragment (4-32) in a sample. That is, the invention has enabled an immunoassay of human BNP fragment (4-32) only in a sample containing BNP and various human BNP fragments. The antibody of the present invention, when combined with other antibodies capable of specifically detecting different fragments, enables immunological measurement of abundance ratios of different fragments, and the invention can contribute to making a disease prognosis using such a ratio or ratios as an index.

### BRIEF DESCRIPTION OF DRAWINGS

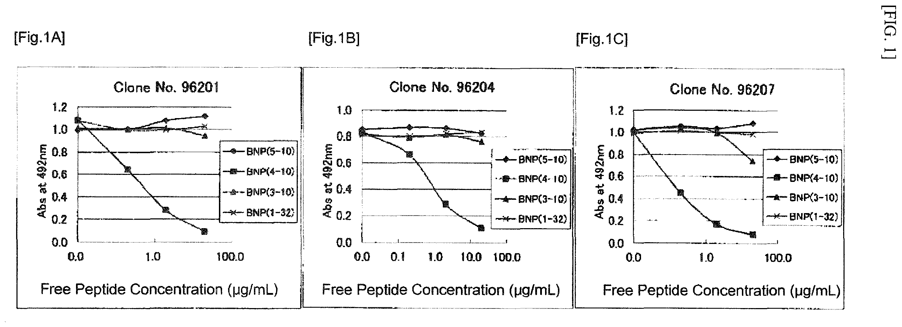

[Fig. 1] FIG. 1 shows graphs representing the results of competitive ELISA confirming the reactivity of three kinds of antibodies of the present invention with synthetic peptides (BNP (1-32), BNP (3-10), BNP (4-10), and BNP (5-10)) of different concentrations (1A: 96201 antibody; 1B: 96204 antibody; 1C: 96207 antibody).

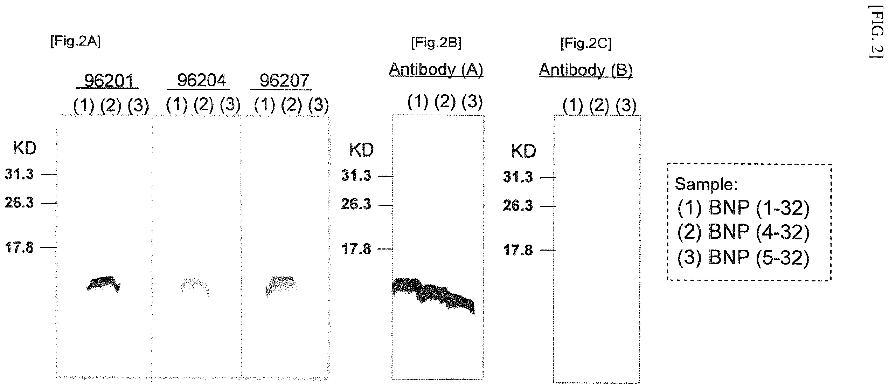

[Fig. 2] FIG. 2 shows electrophoretic patterns representing the result of western blotting confirming the reactivity of different antibodies with synthetic peptides ((1) BNP (1-32), (2) BNP (4-32), and (3) BNP (5-32)) (2A: three kinds of antibodies of the present invention (96201 antibody, 96204 antibody, 96207 antibody); 2B: antibody (A) that recognizes the cyclic portion of human BNP; 2C: IgG antibody (B) as a negative control).

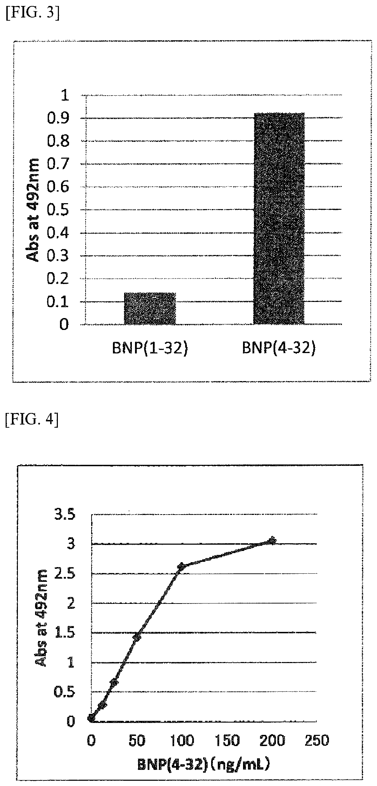

[Fig. 3] FIG. 3 shows a graph representing the result of sandwich ELISA confirming the reactivity of a combination of antibodies with human BNP (1-32) and human BNP fragment (4-32) (primary antibody: 96207 antibody representing an antibody of the present invention; secondary antibody: antibody 33236 that recognizes human BNP (26-32)).
[Fig. 4] FIG. 4 is a graph representing the result of sandwich ELISA confirming the reactivity of a combination of antibodies with human BNP fragment (4-32) at different concentrations of human BNP fragment (4-32) (primary antibody: 96207 antibody representing an antibody of the present invention; secondary antibody: antibody 33236 that recognizes human BNP (26-32)).

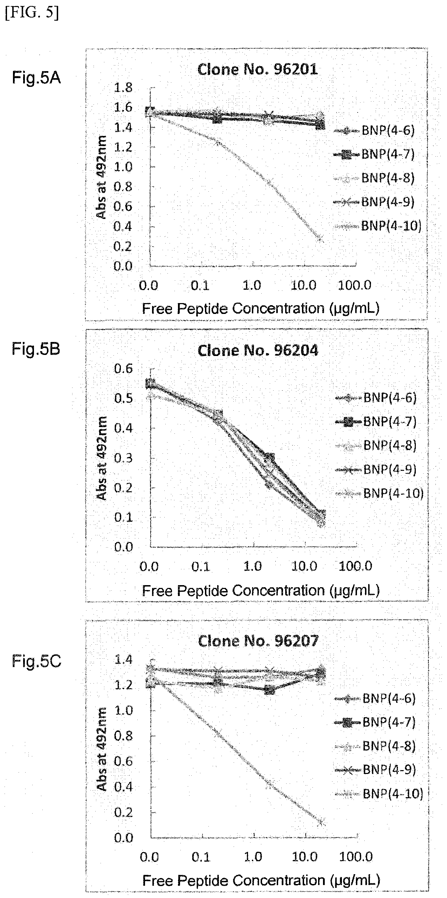

[Fig. 5] FIG. 5 shows graphs representing the results of competitive ELISA confirming the reactivity of three kinds of antibodies of the present invention with different concentrations of synthetic peptides (BNP (4-6), BNP (4-7), BNP (4-8), BNP (4-9), BNP (4-10)) (5A: 96201 antibody; 5B: 96204 antibody; 5C: 96207 antibody).

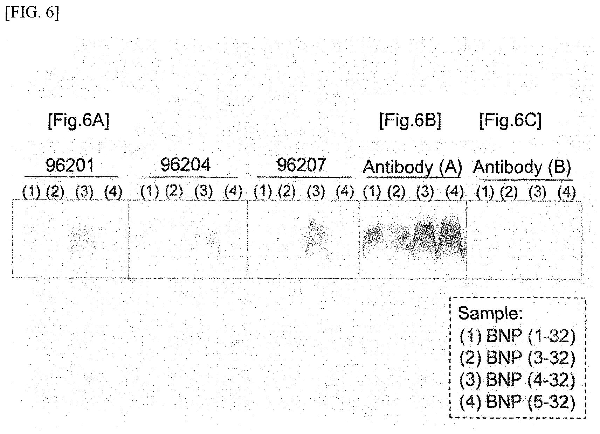

[Fig. 6] FIG. 6 shows electrophoretic patterns representing the result of western blotting confirming the reactivity of different antibodies with synthetic peptides ((1) BNP (1-32), (2) BNP (3-32), (3) BNP (4-32), (3) BNP (5-32)) (6A: three kinds of antibodies of the present invention (96201 antibody, 96204 antibody, 96207 antibody); 6B: antibody (A) that recognizes the cyclic portion of human BNP; 6C: IgG antibody (B) as a negative control).

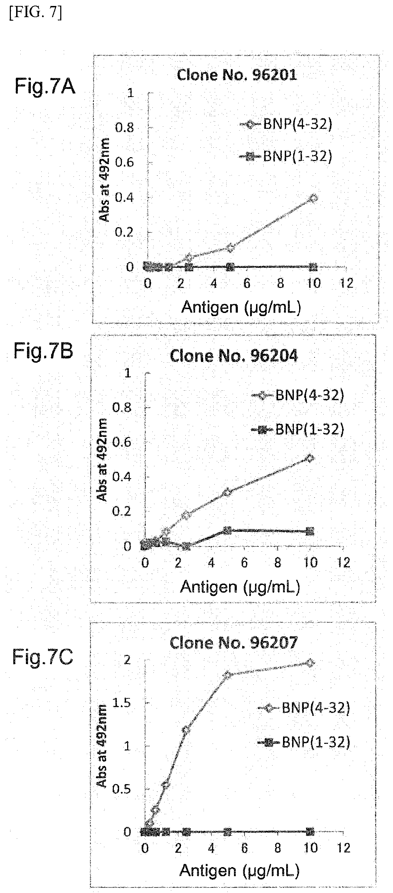

[Fig. 7] FIG. 7 shows graphs representing the results of sandwich ELISA confirming the reactivity of a combination of antibodies against human BNP fragments (4-32) and (1-32) at different concentrations of human BNP fragments (4-32) and (1-32) (7A: primary antibody is a 96201 antibody representing an antibody of the present invention, and secondary antibody is a 33236 antibody that recognizes human BNP (26-32); 7B: primary antibody is a 96204 antibody representing an antibody of the present invention, and secondary antibody is a 33236 antibody that recognizes human BNP (26-32); 7C: primary antibody is a 96207 antibody representing an antibody of the present invention, and secondary antibody is a 33236 antibody that recognizes human BNP (26-32)).

### DESCRIPTION OF EMBODIMENTS

As used herein, the terms "react", "recognize", "bind", and "showing cross-reactivity" used in conjunction with human BNP or human BNP fragments (also referred to collectively as "human BNPs") have the same meaning, and are to be construed in their broadest sense without being bound to these examples. Whether an antibody reacts with antigens (compounds) such as human BNPs can be confirmed by using various methods, including, for example, solid phase-antigen ELISA, western blotting, competitive ELISA, and sandwich ELISA, as described below. It is also possible to use a method that takes advantage of the principle of surface plasmon resonance (SPR method), for example. The SPR method may be carried out using devices, sensors, and reagents commercially available under the trade name Biacore®.

The antibody of the present invention not reacting with a given compound means that the antibody of the present invention is essentially not reactive to the compound. By "essentially not reactive", it means that, for example, the antibody of the present invention does not show augmented reactivity when immobilized and measured by any of the ELISA techniques above. More specifically, "essentially not reactive" means that the reactivity of the antibody against a compound is not significantly different from the reactivity of a control (no compound is added). Evidently, the antibody can be confirmed as being "essentially not reactive" by using other methods and techniques known to a skilled person, aside from ELISA.

In the present invention, human BNP (1-32) is a 32-amino acid human mature B-type natriuretic hormone that includes a 17-amino acid cyclic portion formed by intramolecular disulfide linkage between the cysteine residues present in 7th amino acid and 23rd amino acid. The hormone also includes N- and C-terminal moieties that are 9-amino acid and 6-amino acid long, respectively. The human BNP (1-32) also corresponds to positions 77 to 108 of the amino acid sequence of human B-type natriuretic hormone precursor consisting of 108 amino acids (also referred to as "human proBNP").

The BNP fragment (3-32) of the present invention is a fragment that occurs after processing of two amino acids (indicated by capital letters SP) at the N-terminus of BNP (1-32). The BNP fragment (4-32) is a fragment that occurs after processing of three amino acids (SPK) at the N-terminus of BNP (1-32). The BNP fragment (5-32) is a fragment that occurs after processing of four amino acids (SPKM) at the N-terminus of BNP (1-32). In this specification, the expressions, such as human BNP fragment (4-32) and human BNP (4-32), have the same meaning, unless otherwise specifically stated.

The antibody of the present invention is an antibody that reacts with human BNP fragment (4-32) but does not react with full-length human BNP (1-32). Preferably, the antibody of the present invention is an antibody that reacts with human BNP fragment (4-32) at a site containing the N-terminus, and that reacts with human BNP fragment (4-10). More preferably, the antibody of the present invention is an antibody that does not react with human BNP fragment (3-32) and human BNP fragment (5-32).

The antibody of the present invention may be a polyclonal antibody or a monoclonal antibody, and is preferably a monoclonal antibody.

Specific examples of the monoclonal antibody include monoclonal antibodies produced by hybridomas 96201 (NITE BP-02331), 96204 (NITE BP-02332), and 96207 (NITE BP-02333) (these will be referred to also as 96201 antibody, 96204 antibody, and 96207 antibody, respectively). Monoclonal antibodies that share the same cross-reactivity with 96201, 96204, and 96207 antibodies, or that compete with these antibodies also fall within the range of the antibody of the present invention. The 96201, 96204, and 96207 antibodies include antibodies that can specifically recognize the amino acid sequence (human BNP (4-10)) of the same epitope (antigenic determinant) recognized by the 96201, 96204, and 96207 antibodies. Monoclonal antibodies that compete with the 96201, 96204, and 96207 antibodies include monoclonal antibodies that complete with the 96201, 96204, and 96207 antibodies for human BNP (4-10), and the extent of competitive inhibition is 50% or more, preferably 80% or more, further preferably 90% or more.

The antibody of the present invention may be used in the form of a whole antibody molecule, or as a functional fragment of the antibody having antigen-antibody reaction activity. The antibody of the present invention also may be one obtained through immunization of animals, as above, or by using a genetic recombination technique, or may be a chimeric antibody. Examples of the functional fragment of the antibody include F(ab')2 and Fab', and these may be produced by obtaining the antibody in the manner described above, and treating the antibody with a protease (for example, pepsin, papain).

An antigen (immunogen) synthesized as a peptide containing the N-terminus of human BNP (4-32) is used for the production of the monoclonal antibody of the present invention. The peptide is not particularly limited, as long as it is a peptide containing the N-terminus. For example, the peptide may be a peptide corresponding to the amino acid residues in positions 4 to 26 of human BNP, or may be human BNP fragment (4-32) itself. Specifically, the monoclonal antibody may be produced by dissolving the N-terminus of human BNP (4-32), or, preferably, a peptide corresponding to the amino acid residues in positions 4 to 10 of human BNP (hereinafter, "human BNP (4-10)), in a solvent such as phosphate buffered saline, and administering and immunizing an animal with this solution. For improved immunogenicity, the peptide may, for example, be bound to bovine serum albumin (BSA), ovalbumin (OVA), or keyhole limpet hemocyanin (KLH), and the resulting conjugate may be used for immunization using an emulsion, after adding an appropriate adjuvant to the peptide conjugate as required. The adjuvant may be a common adjuvant such as a water-in-oil emulsion, a water-in-oil-in-water emulsion, an oil-in-water emulsion, a liposome, or an aluminum hydroxide gel, or may be a protein or peptidic substance of biological origin. For example, preferred for use as an adjuvant are incomplete Freund's adjuvants and complete Freund's adjuvants The route, dose, and time of adjuvant administration are not particularly limited. It is, however, preferable that these be appropriately selected so as to boost the desired immune response in an animal immunized with the antigen.

The type of animal to be immunized is not particularly limited. However, preferred are mammals, for example, such as mice, rats, cows, rabbits, goats, and sheep. Mice are more preferred. The animal may be immunized following common techniques. For example, an antigen solution, preferably a mixture with an adjuvant, may be subcutaneously, intradermally, intravenously, or intraperitoneally injected to the animal for immunization. Typically, the immune response is different for different species or lines of animals immunized, and, accordingly, the immunization schedule should be appropriately set according to the type of animal used. Preferably, the antigen is repeatedly administered several times after the first immunization.

This is followed by the following procedures when obtaining a monoclonal antibody. However, the procedures are not limited to the following, and the production of the monoclonal antibody itself may follow, for example, the method described in Antibodies, A Laboratory Manual (Cold Spring Harbor Laboratory Press, (1988)).

Hybridomas can be produced as follows. After the final immunization, antibody-producing cells, specifically, spleen cells or lymph node cells, are removed from the immunized animal, and fused with myeloma cells, which have high proliferative capacity. Preferably, the cells used for cell fusion are cells having high antibody producing capacity (in terms of both quality and quantity), and the myeloma cells are preferably compatible with the animal which the antibody-producing cells to be fused derive from. Cell fusion may be achieved using methods known in the art. For example, the cell may be fused by using the polyethylene glycol method, a method using Sendai virus, or a method using electrical current. The obtained hybridomas may be grown following a known method, and the desired hybridoma can be selected by checking the properties of the antibody it produces. Cloning of the hybridoma can be achieved by using a known method, for example, such as a limiting dilution method or a soft agar method.

Efficient selection of hybridomas is possible by taking into consideration the actual assay conditions of the antibodies produced. For example, hybridomas are obtained by selecting cells producing antibodies that react with human BNP fragments, using a technique such as ELISA, RIA, or western blotting. Specifically, a hybridoma producing a monoclonal antibody that specifically recognizes only human BNP (4-32) can be obtained by western blotting of a culture supernatant of a single hybridoma, using human BNP (1-32), human BNP (4-32), and human BNP (5-32) as detection antigen samples. Alternatively, the hybridoma can be obtained by competitive ELISA, using, for example, human BNP (3-10), human BNP (4-10), human BNP (5-10), human BNP (1-32), human BNP (3-32), human BNP (4-32), and human BNP (5-32).

A monoclonal antibody having desired properties can be produced from a large culture of the hybridoma selected in the manner described above. The method used to grow a large culture of hybridoma is not particularly limited. For example, the hybridoma may be cultured in a suitable medium to produce a monoclonal antibody in the medium, or the hybridoma may be intraperitoneally injected into a mammal, and the cells may be grown to produce antibodies in the ascites fluid. The monoclonal antibody may, for example, be purified by appropriately combining methods such as anion exchange chromatography affinity chromatography, ammonium sulfate fractionation, PEG fractionation, and ethanol fractionation.

In the present invention, the monoclonal antibodies 96201, 96204, and 96207 were obtained in the manner described above. These antibodies react with human BNP (4-10) but do not react with human BNP (3-10) and human BNP (5-10), and thus, are antibodies specific to the site containing the N-terminus of human BNP (4-32), as will be described later in Examples.

The present invention can also provide a method for assaying human BNP (4-32) with an antibody (first monoclonal antibody) specific to human BNP (4-32), and an antibody (second antibody) having a human BNP (4-32) recognition site different from that of the first monoclonal antibody after a specimen is treated with a protein denaturing agent.

The second antibody may be any of an antiserum, a polyclonal antibody and a monoclonal antibody, or may be a chimeric antibody, or an antibody fragment retaining the immunological properties. Preferred are monoclonal antibodies. An example is an anti-human BNP (26-32) monoclonal antibody (Clone #33236) that recognizes human BNP (26-32) obtained through immunization of a mouse with the 7-residue peptide in the C-terminal region of human BNP following a method commonly used by a skilled person for the production of a monoclonal antibody (e.g., the Kohler and Milstein's method; Nature, Vol. 256, p. 495 (1975)). It is also possible to use, for example, a monoclonal antibody that recognizes the cyclic portion of human BNP.

The first or second monoclonal antibody, or both of these antibodies may be used as immobilized (solid-phase) antibodies immobilized on an insoluble support, or as labeled antibodies labeled with a common marker known to a skilled person. For example, an immobilized antibody may be produced by physically adsorbing or chemically binding (optionally via a suitable spacer) the monoclonal antibody to an insoluble support. The insoluble support may be a polymer substrate such as a polystyrene resin, an inorganic substrate such as glass, or a polysaccharide substrate such as cellulose or agarose. The shape of insoluble support is not particularly limited, and may be freely selected. Examples include a plate shape (for example, a microplate or a membrane), beads or fine particles (for example, latex particles, colloidal gold particles), and cylinders (for example, a test tube).

A measurement of human BNP fragment (4-32) in a sample is possible with the use of, for example, a labeled antibody, labeled protein A, or labeled protein G capable of binding to the first or second monoclonal antibody of the present invention. Examples of the marker that can be used to produce the labeled antibody include enzymes, fluorescent substances, chemiluminescent materials, biotin, avidin, radioisotopes, colloidal gold particles, and colored latexes. The method used for binding of the marker and the antibody may be selected from methods available to a skilled person, including, for example, the glutaraldehyde method, the maleimide method, the pyridyl disulfide method, and the periodic acid method. However, the types of immobilized antibodies and labeled antibodies, and the method of production of these antibodies are not limited to the foregoing examples. For example, when enzymes such as peroxidase and alkali phosphatase are used as markers, the enzymatic activity can be measured using a substrate specific to the enzyme (for example, O-phenylenediamine (hereinafter, "OPD") or 3,3',5,5'-tetramethylbenzidine when the enzyme is horseradish peroxidase (hereinafter, "HRP"), or p-nitrophenyl phosphate when the enzyme is ALP). When biotin is used as a marker, at least avidin or enzyme-modified avidin is typically used for reaction.

The term "solid phase" as used herein may describes "insoluble support", and the terms "immobilize", "immobilized", "immobilize on a solid phase", "sensitize", and "adsorb" as used herein may describe supporting an antigen or an antibody on an insoluble support, or an antigen or an antibody being supported on an insoluble support, either physically or chemically. It is also noted that the term "detect" or "assay" is to be construed in their broadest sense, including proving the presence of human BNP fragment (4-32) and/or quantifying human BNP fragment (4-32), and not to be construed in their narrower sense in any ways.

Preferably, the method of the present invention denatures the human BNP fragment, which is an analyte, by the action of a protein denaturing agent. Specifically, the specimen is preferably treated with a protein denaturing agent for assaying the human BNP fragment with an antibody of the present invention that reacts with human BNP fragment (4-32) but does not react with full-length human BNP (1-32).

As used herein, "denature" means to expose at least a part of the protein interior by altering the three-dimensional structure of the BNP fragment. The protein denaturing agent is not particularly limited, as long as it has a protein denaturing effect. Examples include surfactants, chaotropic agents, acids, and organic solvents. The protein denaturing agent may be used by itself, or a plurarity of protein denaturing agents may be used in combination.

Examples of the surfactants include anionic surfactants, cationic surfactants, and nonionic surfactants. Preferred are anionic surfactants, and sodium dodecyl sulfate (SDS) is more preferred.

Examples of the chaotropic agents include urea, and guanidine.

Examples of the acids include inorganic acids and organic acids. Examples of the organic acids include acetic acid, formic acid, propionic acid, butyric acid, isobutyric acid, and mixtures thereof. Preferred is acetic acid.

The protein denaturing agent treatment may be, for example, a treatment at 15°C to 100°C for 1 to 60 minutes.

The protein denaturing agent may be used in appropriately selected amounts that improve the sensitivity of the immunoassay, and the amount can be appropriately selected according to the type of protein denaturing agent used. Typically, the protein denaturing agent is used in an amount of preferably at least 0.01 weight% in terms of the final concentration, and the concentration is preferably 0.1 weight% or less as measured at the time of an immunoassay.

Preferably, the protein denaturing agent exhibits its effect under applied heat. By being heated, the protein denaturing agent is able to easily facilitate protein denaturation. Specifically, the protein denaturing agent is heated at 60°C or more, more preferably about 100°C, for 5 seconds to 20 minutes, more preferably for about 5 to 15 minutes.

The denaturing agent may be used with a reducing agent. Preferred examples of the reducing agent include, but are not limited to, dithiothreitol (DTT), NaBH4, and NaBH₃CN. The reducing agent is used in a concentration of preferably about 0.001 M to 1.0 M in terms of the final concentration. A treatment using the reducing agent may be performed simultaneously with the treatment using the protein denaturing agent, or before the treatment using the protein denaturing agent. In this case, it is preferable that the reducing agent exhibits its effect at room temperature for about 20 minutes to about 1 hour in the above concentration.

A sample to be detected in an assay using the antibody of the present invention may be a bodily fluid of biogenic origin (biological origin). Specific examples include, but are not limited to, blood (whole blood), serum, plasma, urine, saliva, sputum, pancreas extract, tear, ear discharge, and prostatic fluid.

The form of the assay reagent provided by the present invention is not particularly limited, as long as it can be used for assaying human BNP fragment (4-32). The following describes the assay reagent by taking ELISA and immunochromatography as typical examples of labeling immunoassay, and a latex immunoagglutination assay (hereinafter, "LTIA") as a typical example of a particle agglutination immunoassay.

### <Labeling Immunoassay: ELISA>

The assay reagent for the detection of human BNP fragment (4-32) in a sample may include:
(a) a solid phase (e.g., a plate) having a first monoclonal antibody immobilized thereon, and
(b) a second monoclonal antibody labeled with a marker.

The first monoclonal antibody-immobilized solid phase captures human BNP fragment (4-32) in a sample, and forms a human BNP (4-32)-antibody conjugate. The marker-labeled second monoclonal antibody forms a sandwich by reacting with human BNP (4-32)-antibody conjugate, and measurement of human BNP (4-32) in a sample is possible by measuring the amount of the marker using a method that varies with the type of marker used. Specific methods of construction of the assay reagent, for example, immobilization of the first monoclonal antibody on a solid phase, or labeling of the second monoclonal antibody with a marker may be freely selected from methods known to a skilled person, in addition to the methods described in this patent specification. In the foregoing configuration, the assay reagent may be constructed as a homogenous assay system, or, more preferably, a heterogeneous assay system.

Alternatively, the assay reagent maybe opposite of the foregoing configuration, taking into consideration the sensitivity and specificity of the assay reagent, as follows.
(a) a first monoclonal antibody labeled with a marker, and
(b) a solid phase (e.g., a plate) having a second monoclonal antibody immobilized thereon.

In such a configuration, it is preferable to form a human BNP (4-32)-antibody conjugate in advance by mixing a target sample with a solution containing the marker-labeled first monoclonal antibody, and add the solution containing human BNP (4-32)-antibody conjugate to the second monoclonal antibody-immobilized solid phase.

### <Labeling Immunoassay: Immunochromatography>

In a typical configuration of immunochromatography, a target sample solution is allowed to migrate by capillarity through different regions of a sheet-like solid-phase support (e.g., a membrane) along the length of the sheet, continuously from "1. a sample loading site" to "2. a marker reagent site, which is a region of membrane where a marker reagent containing the first monoclonal antibody (the first monoclonal antibody has been labeled with a marker such as colloidal gold particles) is held for development on the membrane", and to "3. a capture reagent site where the second monoclonal antibody for capturing the conjugate of the marker-labeled first monoclonal antibody and human BNP (4-32) has been immobilized".

Specifically, a target sample containing human BNP (4-32) is applied to the sample loading site in a predetermined amount. Once the sample enters the marker reagent site as it spreads and migrates on the solid phase support, human BNP (4-32) binds to the marker reagent (containing the first monoclonal antibody), and forms a human BNP (4-32)-marker reagent conjugate. Human BNP (4-32)-marker reagent conjugate spreads and migrates on the membrane, and enters the capture reagent site, a region of membrane containing the second monoclonal antibody. In the capture reagent site, the capture reagent immobilized on the solid phase support captures the conjugate, and forms a conjugate of the capture reagent (second monoclonal antibody) with human BNP (4-32) and the marker reagent (first monoclonal antibody). The marker reagent can then be detected by an appropriate method (visually detecting an image of colloidal gold particles when colloidal gold particles are used, or detecting a chromogenic reaction by adding a substrate when enzyme is used) to find the presence (or absence) of the analyte.

In this example, for ease of understanding, "1. a sample loading site", and "2. a marker reagent site, which is a region of membrane where a marker reagent containing the first monoclonal antibody (the first monoclonal antibody has been labeled with a marker such as colloidal gold particles) is held for development on the membrane" are independently described in the order of migration of the target sample. However, it will be apparent to a skilled person that other formats and configurations known to a skilled person are also applicable, such as a structure constructed as a stack of the first (1.) and the second (2.) elements.

In the present invention, an assay reagent kit can be provided that includes, for example, a protein denaturing agent in addition to the basic reagent configuration above.

The present invention is described below in greater detail by way of Examples. It is to be noted, however, that the present invention is not limited by the following.

### EXAMPLES

### [Test Example 1] Method of Production of Monoclonal Antibody of the Present Invention

### 1. Preparation of Synthetic Peptides

(1) Human BNP fragment (hereinafter, simply "human BNP") (4-10), human BNP (3-10), and human BNP (5-10)
   Synthesis of these peptides was outsourced.
(2) Human BNP (4-32)
   Synthesis of this peptide was outsourced.

### 2. Other Materials

(1) Complete Freund's adjuvant: Wako Pure Chemical Industries, Ltd; 014-09541
(2) Myeloma cell (SP2/O)
(3) RPMI 1640: GlutaMAX, GIBCO; 61870-036
(4) Fetal Bovine Serum (FBS): Biological Industries; 04-001-1A
(5) Polyethylene glycol solution (PEG): SIGMA, P7306
(6) HAT medium: Cosmo Bio; 16213004
(7) 96-Well plate: NUNC, 167008
(8) HRP-labeled goat anti-mouse IgG(γ) antibody: Southern Biotech; 1030-05

### 3. Preparation of Conjugate Solution for Immunization

A human BNP (4-10) fragment-containing solution (10 mg/ml; dissolved in phosphate buffered saline (PBS)) and a maleimide-activated KLH or OVA solution (10 mg/ml, dissolved in PBS) were mixed in a 1:1 volume ratio, and the mixture was stirred at room temperature for 2 hours to cause reaction. The reaction mixture was then dialyzed against PBS for 2 days at 4°C to obtain a conjugate solution for immunization.

### 4. Production of Anti-Human BNP (4-32) Monoclonal Antibody-Producing Hybridoma

### (1) Immunization of Animal

An emulsion prepared by mixing equal amounts of the conjugate solution for immunization (0.2 to 1 mg/ml) and Complete Freund's adjuvant was injected to female BALB/c mice either subcutaneously or through the paw in a dose of 20 to 30 µg per animal. The emulsion injection was repeated 7 to 8 times at one-week intervals. The antibody titer in the antiserum of the blood collected from the tail vein of each mouse was then measured by solid phase-antigen ELISA, as follows.

### (2) Primary Screening (Solid Phase-Antigen ELISA)

The presence of anti-human BNP (4-10) antibodies in the mouse antiserum was confirmed by ELISA (solid phase-antigen ELISA) by immobilizing a human BNP (4-10)-BSA conjugate solution prepared in the same manner as for the conjugate solution for immunization Details of the solid phase-antigen ELISA is as follows.

### (2-1) Production of Solid Phase-Antigen ELISA Plate

A solution of 1 µg/mL of the human BNP (4-10)-BSA conjugate was prepared by dissolving a solution of this conjugate in a 20 mM phosphate buffer containing 150 mM sodium chloride (pH 7.2; hereinafter, "PBS"). Fifty microliters of the solution was dispensed in each well of a 96-well microplate, and allowed to stand overnight at 4°C.

After washing each well 3 times with 400 µL of PBS containing 0.05% Tween® 20 (hereinafter, "PBST"), 100 µL of PBST containing 1% bovine serum albumin (hereinafter, "BSA-PBST") was added for blocking, which took place at room temperature for 1 hour. This plate was used for ELISA.

### (2-2) Solid Phase-Antigen ELISA

After washing each well of the ELISA plate 3 times with 400 µL of PBST, 50 µL of mouse antiserum diluted 500 to 13,500 times with BSA-PBST was added to each well, and allowed to stand at room temperature for 1 hour. After washing each well 3 times with 400 µL of PBST, 50 µL of HRP-labeled goat anti-mouse IgG(γ) diluted 5,000 times with BSA-PBST was dispensed in each well, and allowed to stand at room temperature for 1 hour. After washing each well 3 times with 400 µL of PBST, 50 µL of citrate buffer (pH 5.0) containing 0.2% o-phenylenediamine and 0.02% hydrogen peroxide was added, and the solution was allowed to stand at room temperature for 10 minutes. The enzymatic reaction was stopped by adding 50 µL of 4.5 N sulfuric acid, and the absorbance at 492 nm wavelength was measured. The spleen or lymph nodes were removed from mice that showed high antibody titers, and spleen-derived cells or lymph node-derived cells were prepared for cell fusion.

### (3) Cell Fusion

The spleen-derived cells or lymph node-derived cells were mixed with myeloma cells in the ratio of 6:1 in terms of number of cells, and these cells were fused by addition of PEG. The fused cells were suspended in HAT medium, and cultured in a CO₂ incubator at 37°C under 5% CO₂ conditions for 8 days to obtain fused cells (hybridomas).

### (4) Screening of Hybridomas (Solid Phase-Antigen ELISA)

The same procedures used in the foregoing solid phase-antigen ELISA were performed, except that a culture supernatant of fused cells was used in place of the mouse antiserum. Wells that showed high absorbance were selected as wells (positive wells) containing anti-human BNP (4-10) antibody-producing hybridomas.

### (5) Secondary Screening (Western Blotting)

The antibody-producing cells selected by primary screening were cultured, and the culture supernatant was screened for anti-BNP (4-32) antibody-producing cells by western blotting in secondary screening.

First, 60 µg/mL solutions of human BNP (1-32), human BNP (4-32), and human BNP (5-32) were prepared by dissolving these peptides in PBS. Each solution was then mixed 1:1 with an SDS process solution containing mercaptoethanol (Cosmo Bio; Prod #423437), and the mixture was processed at 95°C for 10 minutes. Ten microliters of the solution was then added to each lane of Multigel II Mini (Cosmo Bio; Prod #424916), and SDS-PAGE was run following an ordinary method. The electrophoresed gel was placed in the catholyte of a semi-dry blotting reagent, and, after about 10 minutes of shaking, the gel was transferred to a PVDF membrane (Cosmo Bio; Prod #423536) following an ordinary method, using a semidry blotter. After being cut, the PVDF membrane was immersed in BSA-PBST for 1 hour for blocking, and the culture supernatant of the fused cells diluted to a suitable concentration with BSA-PBST was added onto the individual PVDF membranes, which were then allowed to stand at room temperature for 1 hour. After being washed by shaking in PBST, the membrane was placed in HRP-labeled goat anti-mouse IgG(γ) diluted 5,000 times with BSA-PBST, followed by shaking at room temperature for 1 hour. The membrane was washed by shaking in PBST, and immersed in a DAB solution (Dojindo Molecular Technologies, Inc.; Prod #347-00904) that had been diluted to 0.2 mg/mL. After being allowed to stand for 10 minutes, the membrane was transferred into purified water to stop the reaction. After the detection with DAB, anti-human BNP (4-32) antibody-producing positive wells were selected for culture supernatants for which no band was detected when the human BNP (1-32) and the human BNP (5-32) were transfered but for which bands appeared when the human BNP (4-32) was transfered.

### (6) Cloning, and Collection of Monoclonal Antibody

The anti-human BNP (4-32) antibody-producing hybridoma lines selected by primary screening and secondary screening were cloned to produce monoclonal hybridomas, and to purify monoclonal antibodies.

Cloning to produce monoclonal cells followed an ordinary method (limiting dilution), and positive wells were selected by using the same method used in the solid phase-antigen ELISA described above. Finally, three kinds of anti-human BNP (4-32) monoclonal antibody-producing hybridomas were obtained. About 10⁵ cells from each type of hybridoma were administered to the abdominal cavity of a pristane-treated mouse, and the generated ascites fluid was collected from each mouse. The insoluble matter was removed by centrifugation from the collected ascites fluid, and the same amount of saturated ammonium sulfate solution was added. The mixtures were allowed to stand overnight while being stirred, and the precipitate was collected by centrifugation. The precipitate was dissolved in 20 mM Tris buffer (pH 8.0), and dialyzed using the same buffer. The dialysates were adsorbed to separate DEAE-sepharose columns equilibrated with the same buffer, and the IgG fraction obtained by elution in a sodium chloride concentration gradient of 0 to 300 mM in the buffer was dialyzed against 50 mM glycine buffer to obtain three kinds of antibodies.

### [Test Example 2] Epitope Analysis of Monoclonal Antibodies of Present Invention (1)

### 1. Test Method

The epitopes of human BNP (4-32) that react with 96201, 96204, and 96207 antibodies were determined by competitive ELISA, as follows. First, an ELISA plate was created using the same method used for the solid phase-antigen ELISA described above. After washing each well 3 times with 400 µL of PBST, human BNP (1-32), human BNP (3-10), human BNP (4-10), and human BNP (5-10) dissolved in PBS and that had been diluted to 20, 2, and 0.2 µg/mL with BSA-PBST were dispensed on the ELISA plate (25 µL/well).

The 96201, 96204, 96207 antibodies were then dispensed over these wells (25 µL/well) after being diluted to 0.1 µg/mL with BSA-PBST, and were allowed to stand at room temperature for 1 hour. After washing each well 3 times with 400 µL of PBST, 50 µL of HRP-labeled goat anti-mouse IgG(γ) diluted 5,000 times with BSA-PBST was dispensed in each well, and allowed for stand at room temperature for 1 hour. After washing each well 3 times with 400 µL of PBST, 50 µL of citrate buffer (pH 5.0) containing 0.2% o-phenylenediamine and 0.02% hydrogen peroxide was added, and the solution was allowed to stand at room temperature for 10 minutes. The enzymatic reaction was stopped by adding 50 µL of 4.5 N sulfuric acid, and the absorbance at 492 nm wavelength was measured.

### 2. Test Results

The results are shown in FIG. 1. As can be seen in FIG. 1, there was no decrease in absorbance when human BNP (1-32), human BNP (3-10), or human BNP (5-10) was added. On the other hand, the absorbance decreased in a concentration dependent fashion with human BNP (4-10). In this assay system, the absorbance depends on the amount of the antibody that has bound to the human BNP (4-10)-BSA conjugate immobilized on plate. That is, the decrease of fluorescence intensity by addition of human BNP (4-10) indicates that the free human BNP (4-10) in the solution has inhibited the binding of 96201, 96204, and 96207 antibodies to the immobilized conjugate. The test therefore demonstrated that the 96201, 96204, and 96207 antibodies recognize the human BNP (4-10) at a site containing the N-terminus

### [Test Example 3] Epitope Analysis of Monoclonal Antibodies of Present Invention (2)

### 1. Test Method

The epitopes of 96201, 96204, and 96207 antibodies were analyzed by western blotting. For western blotting, the same procedures described above were used except that the following antibodies were used in place of the culture supernatant of the fused cells. Specifically, human BNP (1-32), human BNP (4-32), and human BNP (5-32) were transferred to a PVDF membrane as above, and, after blocking, the antibodies of the present invention (monoclonal antibodies 96201, 96204, and 96207), an antibody (monoclonal antibody (A)) that recognizes the cyclic portion of human BNP, and an antibody (monoclonal antibody (B); negative control) that does not recognize human BNP (1-32), human BNP (4-32), and human BNP (5-32) were added to cut PVDF membranes after diluting these antibodies to 5 µg/mL with BSA-PBST.

### 2. Test Results

The results are shown in FIG. 2. As can be seen in FIG. 2, the 96201, 96204, and 96207 antibodies were shown to specifically react with only human BNP (4-32), and not to react with human BNP (1-32) and human BNP (5-32).

### [Test Example 4] Sandwich ELISA of Human BNP (4-32) and Human BNP (1-32) Using Monoclonal Antibody (1)

### 1. Test Method

The 96207 antibody was diluted to 5 µg/mL with PBS, and this solution was dispensed to each well of a 96-well micro plate in an amount of 50 µL per well. The plate was then allowed to stand overnight at 4°C. After washing each well 3 times with 400 µL of PBST, 100 µL of BSA-PBST was added for blocking, which took place at room temperature for 1 hour. This plate was used for ELISA. After washing each well of the ELISA plate 3 times with 400 µL of PBST, a 50 µg/mL solution of human BNP (1-32) or human BNP (4-32) in PBS was mixed 1:9 with a pH 8.5 processing solution containing 1% SDS (sodium dodecyl sulfate), 50 mM DTT (dithiothreitol), and 0.2 M PB. The mixture was processed at 95°C for 10 minutes, and dispensed in the wells of the ELISA plate in an amount of 50 µL per well after being diluted to 50 ng/mL with BSA-PBST. After washing the ELISA plate 3 times with 400 µL of PBST, a 1 µg/mL solution of biotin-labeled 33236 antibody diluted with BSA-PBST and that recognizes human BNP (26-32) was dispensed in an amount of 50 µL per well, and was allowed for stand at room temperature for 1 hour. After washing the plate 3 times with 400 µL of PBST, a 0.2 µg/mL solution of Immuno Pure® Streptavidin, HRP Conjugated (available from PIERCE; Prod #21126) diluted with BSA-PBST was dispensed in an amount of 50 µL per well, and allowed to stand at room temperature for 30 minutes. After washing each well 3 times with 400 µL of PBST, 50 µL of citrate buffer (pH 5.0) containing 0.2% o-phenylenediamine and 0.02% hydrogen peroxide was added, and the solution was allowed to stand at room temperature for 10 minutes. The enzymatic reaction was stopped by adding 50 µL of 4.5 N sulfuric acid, and the absorbance at 492 nm wavelength was measured.

### 2. Test Results

The result is shown in FIG. 3. As can be seen in FIG. 3, the absorbance was considerably higher when human BNP (4-32) was added than when human BNP (1-32) was added. This result indicates that immobilized 96207 antibody binds to denatured and reduced human BNP (4-32) but does not bind to human BNP (1-32). That is, it was found that 96207 antibody was specific to human BNP (4-32), and that an assay specific to human BNP (4-32) can be set up by using this antibody. It should be noted here that no color signal was produced with human BNP (1-32) or (4-32) when these molecules were not denatured or reduced (the result is not shown in the figure). This suggests that denaturation or reduction has made the antibody binding site of human BNP (4-32) more easily recognizable by the antibody, and that denaturation or reduction is useful for the specific assay of human BNP (4-32).

### [Test Example 5] Sandwich ELISA of Human BNP (4-32) and Human BNP (1-32) Using Monoclonal Antibody (2)

### 1. Test Method

An ELISA plate was made in the same manner as in sandwich ELISA (1) described above. After washing each well 3 times with 400 µL of PBST, a 200 µg/mL solution of human BNP (4-32) in PBS was mixed 1:9 with a pH 8.5 processing solution containing 1% SDS and 0.2 M PB. The mixture was processed at 95°C for 10 minutes, and dispensed on the ELISA plate in an amount of 50 µL per well after being diluted to 200, 100, 50, 25, and 12.5 ng/mL with BSA-PBST.

### 2. Test Results

The result is shown in FIG. 4. As can be seen in FIG. 4, when human BNP (4-32) was added after the treatment with 1% SDS, the absorbance increased in a manner dependent on the concentration of human BNP (4-32). This indicates that increased sensitivity can be obtained with human BNP (4-32) when denatured.

### [Test Example 6] Epitope Analysis of Monoclonal Antibodies of Present Invention (3)

### 1. Test Method

Competitive ELISA was carried out to narrow down the epitope of human BNP (4-32) that reacts with 96201, 96204, and 96207 antibodies. First, an ELISA plate was made using the same method used for the solid phase-antigen ELISA described above. Wells were washed 3 times with 400 µL of PBST. Each of synthetic peptides, human BNP (4-6), (4-7), (4-8), (4-9), or (4-10) was dissolved in PBS (synthesis of these peptides was outsourced) and dispensed on the ELISA plate in an amount of 25 µL per well after diluting to 20, 2, and 0.2 µg/mL with BSA-PBST. The subsequent procedures followed the method described in Test Example 2: Epitope Analysis of Monoclonal Antibodies of Present Invention (1).

### 2. Test Results

The results are shown in FIG. 5. As can be seen in FIGS. 5A and 5C, a decrease in absorbance was not observed with 96201 and 96207 antibodies when human BNP (4-6), human BNP (4-7), human BNP (4-8), or human BNP (4-9) was added. However, the absorbance decreased in a concentration dependent fashion only when human BNP (4-10) was added. This result demonstrates that 96201 and 96207 antibodies recognize the site containing the first seven amino acids from the N-terminus of human BNP (4-32). On the other hand, as shown in FIG. 5B, the 96204 antibody showed a concentration-dependent decrease of absorbance, regardless of whether which peptide antigen was added, demonstrating that 96204 antibody recognizes the site containing the first three amino acids from the N-terminus of human BNP (4-32).

### [Test Example 7] Epitope Analysis of Monoclonal Antibodies of Present Invention (4)

### 1. Test Method

A further analysis was conducted by western blotting with regard to the epitopes of 96201, 96204, and 96207 antibodies. Specifically, the same procedures used in Test Example 3: Epitope Analysis of Monoclonal Antibodies of Present Invention (2) were used, except that the analysis used human BNP (1-32), human BNP (3-32), human BNP (4-32), and human BNP (5-32).

### 2. Test Results

The results are shown in FIG. 6. As can be seen in FIG. 6, 96201, 96204, and 96207 antibodies were shown to specifically react only with human BNP (4-32), but not with human BNP (1-32), BNP (3-32), and human BNP (5-32).

### [Test Example 8] Sandwich ELISA of Human BNP (4-32) and Human BNP (1-32) Using Monoclonal Antibody (3)

### 1. Test Method

First, 96201, 96204, and 96207 antibodies were diluted to 5 µg/mL in PBS. Separately, an antibody (mouse monoclonal antibody (C)) that does not react with human BNP (1-32) and human BNP (4-32) was diluted to 5 µg/mL with PBS. These solutions were each dispensed in the wells of a 96-well microplate in an amount of 50 µL per well, and were allowed to stand overnight at 4°C. After washing each well 3 times with 400 µL of PBST, 100 µL of BSA-PBST was added for blocking, which took place at room temperature for 1 hour. This plate was used for ELISA. Wells of the ELISA plate were washed 3 times with 400 µL of PBST. Human BNP (1-32) or human BNP (4-32) was diluted to the concentrations of 10, 5, 2.5, 1.25, 0.63, 0.31, and 0.16 µg/mL with BSA-PBST and dispensed in each well of the ELISA plate in an amount of 50 µL per well. After washing the ELISA plate 3 times with 400 µL of PBST, a biotin-labeled 33236 antibody (2 µg/mL) that recognizes human BNP (26-32) was dispensed in an amount of 50 µL per well, and allowed to stand at room temperature for 1 hour. After washing the plate 3 times with 400 µL of PBST, Immuno Pure® Streptavidin, HRP Conjugated (PIERCE; Prod #21126) (0.2 µg/mL) was dispensed in an amount of 50 µL per well, and allowed to stand at room temperature for 30 minutes. After washing each well 3 times with 400 µL of PBST, 50 µL of citrate buffer (pH 5.0) containing 0.2% o-phenylenediamine and 0.02% hydrogen peroxide was added, and the solution was allowed to stand at room temperature for 10 minutes. The enzymatic reaction was stopped by adding 50 µL of 4.5 N sulfuric acid, and the absorbance at 492 nm wavelength was measured. The absorbance obtained from the mouse monoclonal antibody (C) was subtracted from the absorbance obtained from 96201, 96204, or 96207 antibody, and this value was determined as the absorbance due to reactivity of 96201, 96204, or 96207 antibody.

### 2. Test Results

The results are shown in FIG. 7. As shown in FIG. 7, the absorbance was considerably higher when human BNP (4-32) was added than when human BNP (1-32) was added, most notably with 96207 antibody. In human BNP (4-32), the reactivity increased in a concentration dependent fashion, demonstrating that an assay specific to human BNP (4-32) can be established. In this Test Example, human BNP (4-32) was not denatured or reduced, and the antigen to be reacted was used in larger amounts than in Test Examples 4 and 5. The results therefore suggest that the binding of antibody is not completely blocked even in the absence of denaturation or reduction, though the antibody can more easily recognize the antibody binding site of human BNP (4-32) when the antigen is denatured or reduced. The lack of any previous report of the acquisition of antibodies against human BNP (4-32) is probably due to an oversight of this finding.

### INDUSTRIAL APPLICABILITY

The antibody of the present invention is an antibody that reacts with human BNP fragment (4-32) but does not react with full-length human BNP (1-32), and enables an immunological assay specific to human BNP fragment (4-32) in a sample. That is, the invention enables an immunological assay of only human BNP fragment (4-32) even in a sample containing BNP and a variety of human BNP fragments.

### REFERENCE TO DEPOSITED BIOLOGICAL MATERIAL

(1) Hybridoma 96201 Producing Antibody Number 96201
   (i) Name and Address of Depositary at Which the Biological Material is Deposited
      National Institute of Technology and Evaluation
      Kazusa Kamatari 2-5-8, Kisarazu-shi, Chiba, Japan, 292-0818
   (ii) Date of Which the Biological Material was Deposited at the Depositary Identified in (i)
      August 18, 2016
   (iii) Accession Number Assigned to the Deposited Material by the Depositary Identified in (i)
      NITE BP-02331
(2) Hybridoma 96204 Producing Antibody Number 96204
   (i) Name and Address of Depositary at which the Biological Material is Deposited
      National Institute of Technology and Evaluation
      Kazusa Kamatari 2-5-8, Kisarazu-shi, Chiba, Japan, 292-0818
   (ii) Date of Which the Biological Material was Deposited at the Depositary Identified in (i)
      August 18, 2016
   (iii) Accession Number Assigned to the Deposited Material by the Depositary Identified in (i)
      NITE BP-02332
(3) Hybridoma 96207 Producing Antibody Number 96207
   (i) Name and Address of Depositary at which the Biological Material is Deposited
      National Institute of Technology and Evaluation
      Kazusa Kamatari 2-5-8, Kisarazu-shi, Chiba, Japan, 292-0818
   (ii) Date of Which the Biological Material was Deposited at the Depositary Identified in (i)
      August 18, 2016
   (iii) Accession Number Assigned to the Deposited Material by the Depositary Identified in (i)
      NITE BP-02333

## Claims

1. An immunoassay method for human BNP fragment (4-32) in a sample, wherein the method uses an antibody that reacts with human BNP fragment (4-32) but does not react with full-length human BNP (1-32), human BNP fragment (3-32), and human BNP fragment (5-32).

2. The immunoassay method according to claim 1, wherein the antibody that reacts with human BNP fragment (4-32) but does not react with full-length human BNP (1-32), human BNP fragment (3-32), and human BNP fragment (5-32) is an antibody that reacts with human BNP fragment (4-32) at a site containing the N-terminus.

3. The immunoassay method according to claim 1 or 2, wherein the antibody that reacts with human BNP fragment (4-32) but does not react with full-length human BNP (1-32), human BNP fragment (3-32), and human BNP fragment (5-32) is an antibody that reacts with human BNP fragment (4-10).

4. The immunoassay method according to any one of claims 1 to 3, wherein the antibody that reacts with human BNP fragment (4-32) but does not react with full-length human BNP (1-32), human BNP fragment (3-32), and human BNP fragment (5-32) is a monoclonal antibody.

5. An immunoassay reagent for human BNP fragment (4-32), wherein the immunoassay reagent uses an antibody that reacts with human BNP fragment (4-32) but does not react with full-length human BNP (1-32), human BNP fragment (3-32), and human BNP fragment (5-32).

6. The immunoassay reagent according to claim 5, wherein the antibody that reacts with human BNP fragment (4-32) but does not react with full-length human BNP (1-32), human BNP fragment (3-32), and human BNP fragment (5-32) is an antibody that reacts with human BNP fragment (4-32) at a site containing the N-terminus.

7. The immunoassay reagent according to claim 5 or 6, wherein the antibody that reacts with human BNP fragment (4-32) but does not react with full-length human BNP (1-32), human BNP fragment (3-32), and human BNP fragment (5-32) is an antibody that reacts with human BNP fragment (4-10).

8. The immunoassay reagent according to any one of claims 5 to 7, wherein the antibody that reacts with human BNP fragment (4-32) but does not react with full-length human BNP (1-32), human BNP fragment (3-32), and human BNP fragment (5-32) is a monoclonal antibody.

9. An antibody that reacts with human BNP fragment (4-32) but does not react with full-length human BNP (1-32), human BNP fragment (3-32), and human BNP fragment (5-32).

10. The antibody according to claim 9, which reacts with human BNP fragment (4-32) at a site containing the N-terminus.

11. The antibody according to claim 9 or 10, which reacts with human BNP fragment (4-10).

12. The antibody according to any one of claims 9 to 11, which is a monoclonal antibody.

## Patentansprüche

1. Immunassayverfahren für humanes BNP-Fragment (4-32) in einer Probe, wobei das Verfahren einen Antikörper verwendet, der mit humanem BNP-Fragment (4-32) reagiert, jedoch nicht mit humanem BNP voller Länge (1-32), humanem BNP-Fragment (3-32) und humanem BNP-Fragment (5-32) reagiert.

2. Immunassayverfahren gemäß Anspruch 1, wobei der Antikörper, der mit humanem BNP-Fragment (4-32) reagiert, jedoch nicht mit humanem BNP voller Länge (1-32), humanem BNP-Fragment (3-32) und humanem BNP-Fragment (5-32) reagiert, ein Antikörper ist, der mit humanem BNP-Fragment (4-32) an einer Stelle reagiert, die den N-Terminus enthält.

3. Immunassayverfahren gemäß Anspruch 1 oder 2, wobei der Antikörper, der mit humanem BNP-Fragment (4-32) reagiert, jedoch nicht mit humanem BNP voller Länge (1-32), humanem BNP-Fragment (3-32) und humanem BNP-Fragment (5-32) reagiert, ein Antikörper ist, der mit humanem BNP-Fragment (4-10) reagiert.

4. Immunassayverfahren gemäß irgendeinem der Ansprüche 1 bis 3, wobei der Antikörper, der mit humanem BNP-Fragment (4-32) reagiert, jedoch nicht mit humanem BNP voller Länge (1-32), humanem BNP-Fragment (3-32) und humanem BNP-Fragment (5-32) reagiert, ein monoklonaler Antikörper ist.

5. Immunassayreagens für humanes BNP-Fragment (4-32), wobei das Immunassayreagens einen Antikörper verwendet, der mit humanem BNP-Fragment (4-32) reagiert, jedoch nicht mit humanem BNP voller Länge (1-32), humanem BNP-Fragment (3-32) und humanem BNP-Fragment (5-32) reagiert.

6. Immunassayreagens gemäß Anspruch 5, wobei der Antikörper, der mit humanem BNP-Fragment (4-32) reagiert, jedoch nicht mit humanem BNP voller Länge (1-32), humanem BNP-Fragment (3-32) und humanem BNP-Fragment (5-32) reagiert, ein Antikörper ist, der mit humanem BNP-Fragment (4-32) an einer Stelle reagiert, die den N-Terminus enthält.

7. Immunassayreagens gemäß Anspruch 5 oder 6, wobei der Antikörper, der mit humanem BNP-Fragment (4-32) reagiert, jedoch nicht mit humanem BNP voller Länge (1-32), humanem BNP-Fragment (3-32) und humanem BNP-Fragment (5-32) reagiert, ein Antikörper ist, der mit humanem BNP-Fragment (4-10) reagiert.

8. Immunassayreagens gemäß irgendeinem der Ansprüche 5 bis 7, wobei der Antikörper, der mit humanem BNP-Fragment (4-32) reagiert, jedoch nicht mit humanem BNP voller Länge (1-32), humanem BNP-Fragment (3-32) und humanem BNP-Fragment (5-32) reagiert, ein monoklonaler Antikörper ist.

9. Antikörper, der mit humanem BNP-Fragment (4-32) reagiert, jedoch nicht mit humanem BNP voller Länge (1-32), humanem BNP-Fragment (3-32) und humanem BNP-Fragment (5-32) reagiert.

10. Antikörper gemäß Anspruch 9, der mit humanem BNP-Fragment (4-32) an einer Stelle reagiert, die den N-Terminus enthält.

11. Antikörper gemäß Anspruch 9 oder 10, der mit humanem BNP-Fragment (4-10) reagiert.

12. Antikörper gemäß irgendeinem der Ansprüche 9 bis 11, welcher ein monoklonaler Antikörper ist.

## Revendications

1. Procédé d'immunoessai pour un fragment de BNP humain (4-32) dans un échantillon, dans lequel le procédé utilise un anticorps qui réagit avec un fragment de BNP humain (4-32) mais ne réagit pas avec un BNP humain pleine longueur (1-32), un fragment de BNP humain (3-32), et un fragment de BNP humain (5-32).

2. Procédé d'immunoessai selon la revendication 1, dans lequel l'anticorps qui réagit avec un fragment de BNP humain (4-32) mais ne réagit pas avec un BNP humain pleine longueur (1-32), un fragment de BNP humain (3-32), et un fragment de BNP humain (5-32) est un anticorps qui réagit avec un fragment de BNP humain (4-32) au niveau d'un site contenant l'extrémité N-terminale.

3. Procédé d'immunoessai selon la revendication 1 ou 2, dans lequel l'anticorps qui réagit avec un fragment de BNP humain (4-32) mais ne réagit pas avec un BNP humain pleine longueur (1-32), un fragment de BNP humain (3-32), et un fragment de BNP humain (5-32) est un anticorps qui réagit avec un fragment de BNP humain (4-10).

4. Procédé d'immunoessai selon l'une quelconque des revendications 1 à 3, dans lequel l'anticorps qui réagit avec un fragment de BNP humain (4-32) mais ne réagit pas avec un BNP humain pleine longueur (1-32), un fragment de BNP humain (3-32), et un fragment de BNP humain (5-32) est un anticorps monoclonal.

5. Réactif d'immunoessai pour un fragment de BNP humain (4-32), dans lequel le réactif d'immunoessai utilise un anticorps qui réagit avec un fragment de BNP humain (4-32) mais ne réagit pas avec un BNP humain pleine longueur (1-32), un fragment de BNP humain (3-32), et un fragment de BNP humain (5-32).

6. Réactif d'immunoessai selon la revendication 5, dans lequel l'anticorps qui réagit avec un fragment de BNP humain (4-32) mais ne réagit pas avec un BNP humain pleine longueur (1-32), un fragment de BNP humain (3-32), et un fragment de BNP humain (5-32) est un anticorps qui réagit avec un fragment de BNP humain (4-32) au niveau d'un site contenant l'extrémité N-terminale.

7. Réactif d'immunoessai selon la revendication 5 ou 6, dans lequel l'anticorps qui réagit avec un fragment de BNP humain (4-32) mais ne réagit pas avec un BNP humain pleine longueur (1-32), un fragment de BNP humain (3-32), et un fragment de BNP humain (5-32) est un anticorps qui réagit avec un fragment de BNP humain (4-10).

8. Réactif d'immunoessai selon l'une quelconque des revendications 5 à 7, dans lequel l'anticorps qui réagit avec un fragment de BNP humain (4-32) mais ne réagit pas avec un BNP humain pleine longueur (1-32), un fragment de BNP humain (3-32), et un fragment de BNP humain (5-32) est un anticorps monoclonal.

9. Anticorps qui réagit avec un fragment de BNP humain (4-32) mais ne réagit pas avec un BNP humain pleine longueur (1-32), un fragment de BNP humain (3-32), et un fragment de BNP humain (5-32).

10. Anticorps selon la revendication 9, qui réagit avec un fragment de BNP humain (4-32) au niveau d'un site contenant l'extrémité N-terminale.

11. Anticorps selon la revendication 9 ou 10, qui réagit avec un fragment de BNP humain (4-10).

12. Anticorps selon l'une quelconque des revendications 9 à 11, qui est un anticorps monoclonal.
